# EUROPÄISCHE PATENTSCHRIFT

(11) **EP 0 654 255 B1**
(45) Veröffentlichungstag und Bekanntmachung des Hinweises auf die Patenterteilung: **08.07.1998**
(21) Anmeldenummer: 93810806.5
(22) Anmeldetag: 22.11.1993
(51) Int. Cl.: A61F 2/34

(54) **Innenschale für eine Hüftgelenkpfanne**
Inner socket for acetabular prosthesis
Cupule intérieure pour prothèse d'articulation cotyloidienne de la hanche

(43) Veröffentlichungstag der Anmeldung: 24.05.1995
(73) Patentinhaber: Sulzer Orthopädie AG, 6340 Baar (CH)
(72) Erfinder: Roland, Willi, CH-8413 Neftenbach (CH)
(74) Vertreter: Heinen, Detlef

(56) Entgegenhaltungen:
- EP-A- 0 265 712
- EP-A- 0 313 762
- EP-A- 0 436 317
- EP-A- 0 488 943
- WO-A-93/16662
- DE-A- 3 331 191
- DE-A- 3 533 432
- US-A- 5 092 897

## Beschreibung

Die Erfindung bezieht sich auf eine Innenschale für eine künstliche Hüftgelenkpfanne gemäss dem Oberbegriff von Anspruch 1. Weiter bezieht sich die Erfindung auf ein Verfahren, das erlaubt, die Innenschale mit einer bekannten Aussenschale zu einer Hüftgelenkpfanne zusammenzusetzen. Weiter bezieht sich die Erfindung auf eine Hüftgelenkpfanne mit einer erfindungsgemässen Innenschale.

Künstliche Hüftgelenkpfannen weisen häufig einen zweiteiligen Aufbau auf, einen im Knochen fixierbaren Verankerungskörper beziehungsweise eine Aussenschale und einen Pfannenkörper beziehungsweise eine Innenschale für die Aufnahme eines Gelenkkopfes.

So ist zum Beispiel aus der EP 0 313 762, A1 eine zweiteilige Hüftgelenkpfanne bekannt, die aus einem im Becken mit Hilfe von Knochenschrauben fixierbaren Verankerungskörper in Form einer Halbkugelschale besteht, sowie einem Pfannenkörper, in dessen Pfannenschale ein Gelenkkopf zu liegen kommt. Dabei ist der aus Kunststoff ausgeführte Pfannenkörper mittels eines Schnappverschlusses im Verankerungskörper fixierbar. Pfannenkörper aus Kunststoff haben den Vorteil, dass sie aufgrund der hohen Elastizität des Kunststoffes das federnde Glied einer Schnappverbindung bilden können. Sie weisen aber den Nachteil auf, dass sich die Pfannenschale durch die Einwirkungen des Gelenkkopfes langfristig abnützt, sodass es bei einer Reoperation erforderlich sein kann, den Pfannenkörper oder die gesamte Küftgelenkpfanne zu ersetzen.

Der Erfindung liegt die Aufgabe zugrunde, eine Innenschale mit verminderten Abnutzungseigenschaften zu bilden, die sich auch in einen bestehenden Verankerungskörper, der ursprünglich für die Aufnahme einer Innenschale aus Kunststoff konzipiert wurde, einsetzten lässt.

Diese Aufgabe wird erfindungsgemäss durch die kennzeichnenden Merkmale von Anspruch 1 gelöst. Die Unteransprüche 2 bis 9 beziehen sich auf weitere, vorteilhafte Ausbildungen. Weiter wird die Aufgabe durch ein Verfahren gemäss Anspruch 10 und 11 gelöst.

Die erfindungsgemässe metallische Innenschale ist derart ausgestaltet, dass sie sich in einen bestehenden Verankerungskörper beziehungsweise in eine bestehende Aussenschale einsetzen lässt. Die erfindungsgemässe Innenschale weist den Vorteil auf, dass bei einer Reoperation die Innenschale aus Kunststoff durch eine Innenschale aus Metall ersetzbar ist, derart, dass die eingewachsene Aussenschale an ihrem Ort verbleibt. Somit lässt sich auf eine für das Knochengewebe schonende Weise der Verschleiss der Innenschale verringern. Bestehende metallische Aussenschalen sind im Bereich des Schnappverschlusses in den für Kunststoffinnenschalen üblichen, gröberen Toleranzen gefertigt. Ein weiterer Vorteil der Erfindung ist darin zu sehen, dass Aussenschalen, insbesondere bestehende Aussenschalen, wahlweise mit einer Innenschale aus Kunststoff oder einer Innenschale aus Metall bestückbar sind.

Im folgenden wird die Erfindung anhand von Ausführungsbeispielen im Zusammenhang mit den folgenden Figuren näher erläutert. Es zeigen:
- Fig. 1a: eine Untenansicht einer metallischen Innenschale;
- Fig. 1b: eine Seitenansicht eines Schnittes (B-B) durch die metallische Innenschale;
- Fig. 1c: einen Ausschnitt eines Abstützbereiches;
- Fig. 2a: einen Schnitt durch eine metallische Aussenschale;
- Fig. 2b: einen Ausschnitt einer Nut der Aussenschale;
- Fig. 3a: eine teilweise im Schnitt dargestellte Seitenansicht eines Befestigungselementes;
- Fig. 3b: eine Seitenansicht eines weiteren Befestigungselementes;
- Fig. 3c: eine Aufsicht auf den Kopf eines weiteren Befestigungselementes;
- Fig. 4: ein Detail eines Schnittes durch eine Hüftgelenkpfanne, deren Innen- und Aussenschale durch ein Befestigungselement zusammengehalten sind;
- Fig. 5: einen Ausschnitt eines Schnittes durch eine Hüftgelenkpfanne mit Innen- und Aussenschale und einer weiteren Befestigungsmöglichkeit.

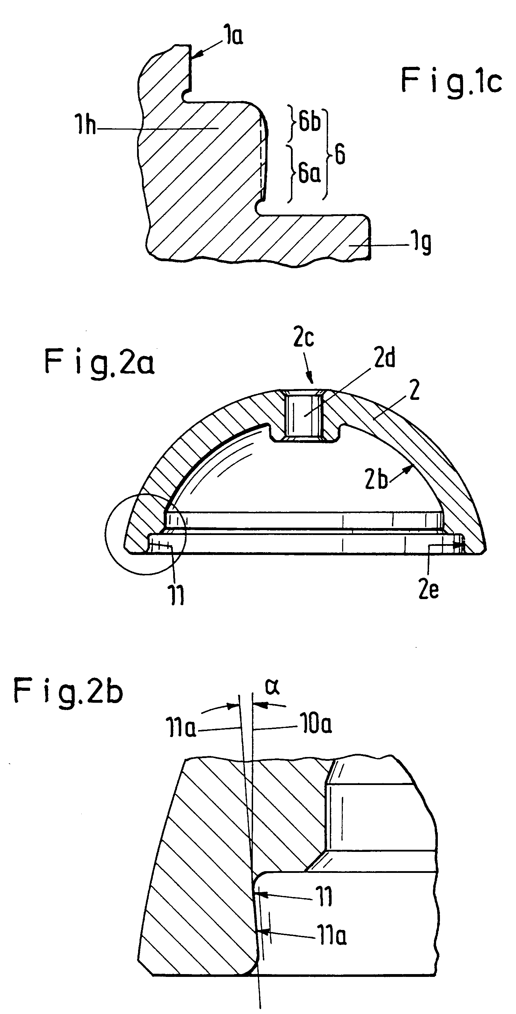

In Fig. 2a ist ein Beispiel einer bekannten, metallischen Aussenschale 2 dargestellt, wie sie für zweiteilige Hüftgelenkpfannen Verwendung findet. Die dargestellte Aussenschale 2 weist im Polbereich 2c eine Ausnehmung 2d auf sowie an der Innenfläche 2b, entlang dem äquatorialen Umfang 2e, eine Nut 11. Der in Fig. 2b dargestellte Ausschnitt aus Fig. 2a zeigt die Nut 11, deren durch den äquatorialen Umfang 2e gebildete Begrenzungsfläche 11a um einen Winkel α zu einer Vertikalen 10 geneigt ist, derart, dass sich der Durchmesser der Nut 11 gegen den Polbereich 2c hin entsprechend dem Winkel α erweitert. Es ist zum Beispiel aus der EP 0 313 762 bekannt, in eine derart gestaltete Aussenschale 2 eine Innenschale 1 aus Kunststoff einzusetzen. Dabei weist die Innenschale 1 an deren Aussenfläche 1a einen ringförmigen Ansatz 1h auf, der in die Nut 11 im Innenhohlraum der Aussenschale 2 einschnappt, sodass die Innenschale 1 in der Aussenschale 2 fixiert ist.

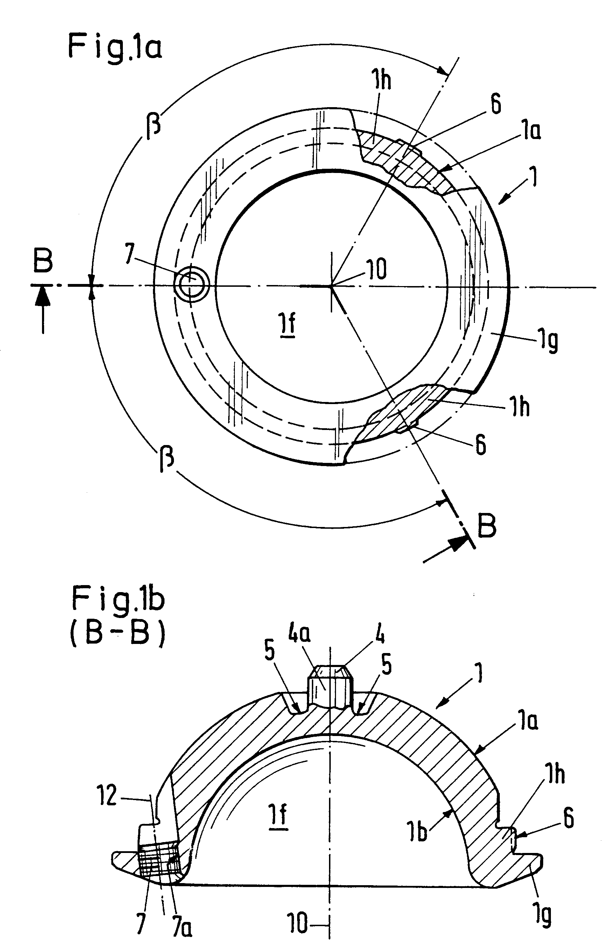

Fig. 1a zeigt eine Untenansicht einer erfindungsgemässen, metallischen Innenschale 1 mit Innenhohlraum 1f, Vorsprung 1g und Aussenfläche 1a. Weiter ist die durch den Pol 4 der Innenschale 1 verlaufende Achsrichtung 10 dargestellt, die in der dargestellten Ansicht vertikal zur Ansichtsebene verläuft. An einer Stelle ist der Vorsprung 1g mit einer Durchbrechung 7, zum Beispiel einer Bohrung, durchbrochen. Weiter weist die Innenschale 1 an deren Aussenfläche 1a, vorzugsweise im Bereich des Äquators, mindestens zwei Abstützbereiche 6 auf, die die Eigenschaft aufweisen, dass sie ein wenig über die Aussenfläche 1a vorstehen. Im dargestellten Ausführungsbeispiel sind zwei Abstützbereiche 6 dargestellt, die, bezüglich einer Verbindungslinie zwischen Achsrichtung 10 und Durchbrechung 7, je um einen Winkel β versetzt im Bereich des Äquators an der Aussenfläche 1a angeordnet sind.

In Fig. 1b ist ein Schitt durch die Innenschale 1 entlang der in Fig. 1a dargestellten Linie B-B gezeigt. Daraus ist die Innenschschale 1 mit Aussenfläche 1a, Innenfläche 1b, dem Innenhohlraum 1f sowie dem Vorsprung 1g ersichtlich. Die Achsrichtung 10 verläuft durch den Pol 4 der Innenschale 1, wobei der Pol 4 im vorliegenden Ausführungsbeispiel als Zentrierzapfen 4a ausgebildet ist, der in einen Abstützbereich 5 mündet und von diesem umgeben ist. Weiter ist die Verlaufsrichtung 12 der Durchbrechung 7 ersichtlich, wobei die Durchbrechung 7 im vorliegenden Ausführungsbeispiel als Bohrung mit Innengewinde 7a ausgeführt ist. Weiter ist der im Bereich des Äquators an der Aussenfläche 1a umlaufende Ansatz 1h der Innenschale 1 ersichtlich. An diesem Ansatz 1h ist ein Abstützbereich 6 derart angeordnet, dass der Abstützbereich 6 radial zur Achsrichtung 10 leicht über den Ansatz 1h vorsteht.

Fig. 1c zeigt den im Bereich des Ansatzes 1h angeordneten Abstützbereich 6 im Detail. Ein radial zur Achsrichtung 10 vorstehender Bereich kann durch eine Viehlzahl unterschiedlicher Ausführungsformen erreicht werden. Im vorliegenden Ausführungsbeispiel weist der Abstützbereich 6 einen Erweiterungsbereich 6a auf, innerhalb dessen sich der Aussendurchmesser des Abstützbereiches 6 zum Pol 4 hin erweitert. Der Erweiterungsbereich 6a geht fliessend über in einen Verengungsbereich 6b, innerhalb dessen der Aussendurchmesser des Abstützbereiches 6 zum Pol 4 hin abnimmt. In achsialer Richtung 10 ist die Höhe des Abstützbereiches 6 durch die Höhe des Ansatzes 1h begrenzt.

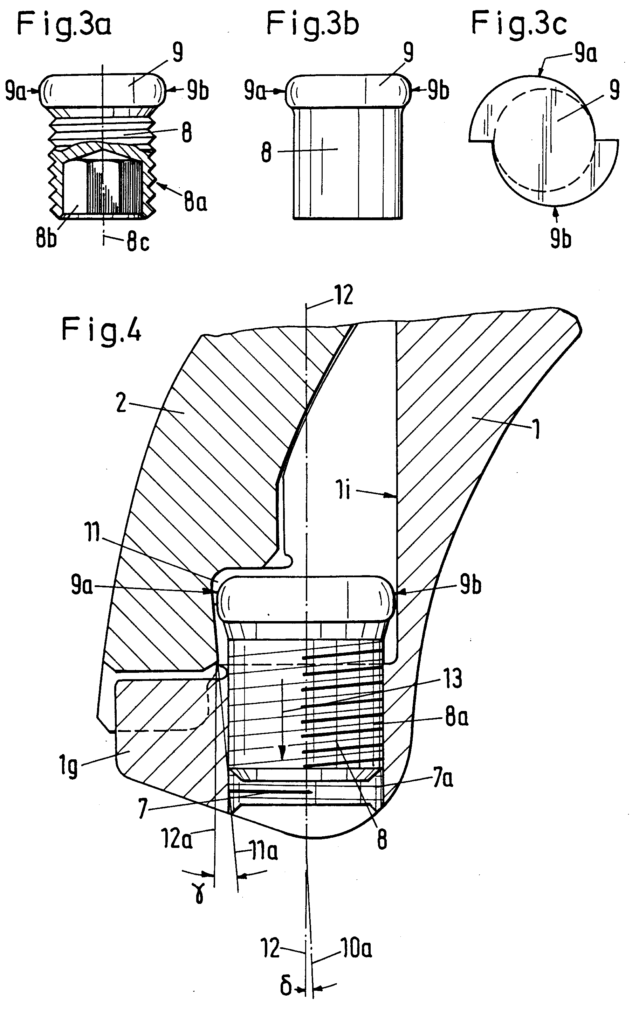

Fig. 3a zeigt ein Befestigungselement 8, das dazu dient die Innenschale 1 in der Aussenschale 2 lösbar zu befestigen. Das zylinderförmige Befestigungselement 8 weist an der Mantelfläche ein Aussengewinde 8a auf, an der einen Stirnseite eine Ausnehmung 8b zum Eingriff mit einem Befestigungswerkzeug und an der anderen Stirnseite einen Kopf 9, der ein gegenüber dem Aussengewinde 8a vergrösserten Durchmesser aufweist. Der Kopf 9 weist parallel zur Achsrichtung 8c des Befestigungselementes 8 verlaufende Kraftübertragungsbereiche 9a, 9b auf, die dazu dienen insbesondere radial zur Achsrichtung 8c auf den Kopf 9 einwirkende Kräfte zu übertragen. Das in Fig. 3b dargestellte Befestigungselement 8 weist im Vergleich zu dem Ausführungsbeispiel gemäss Fig. 3a kein Aussengewinde 8a auf, weshalb sich das Befestigungselement 8 dazu eignet eingeschlagen zu werden. In Fig. 3c zeigt ein weiteres Ausführungsbeispiel eine Aufsicht auf einen Kopf 9, der zwei sich kontinuierlich erweiternde Kraftübertragungsbereiche 9a, 9b aufweist. Das Befestigungselement 8 und der Kopf 9 können auch getrennt ausgeführt sein, zum Beispiel derart, dass der Kopf 9 drehbar im Befestigungselement 8 gelagert ist. Dabei kann der Kopf 9 aus der Aufsicht auch eine rechteckige oder quadratische Form aufweisen.

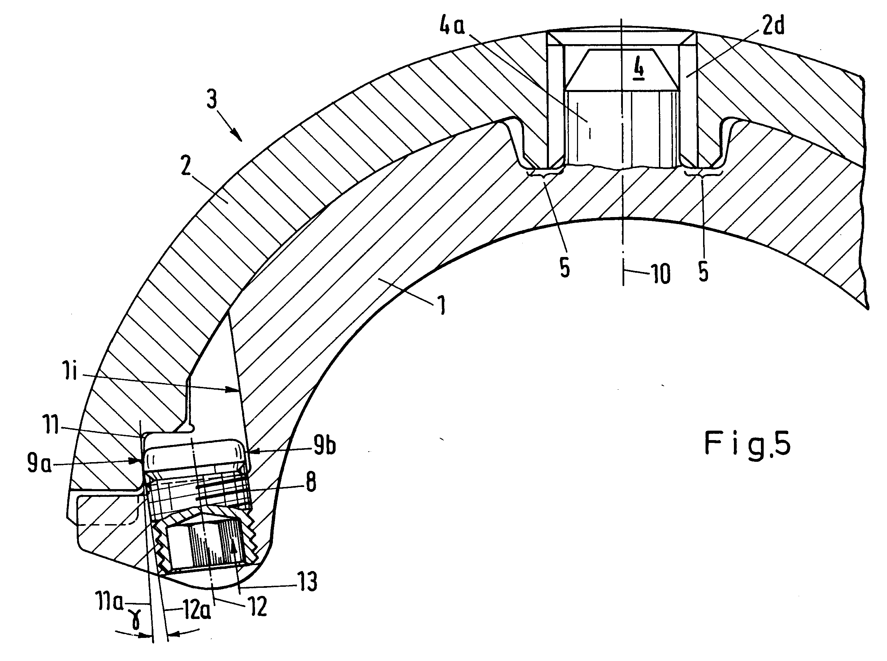

Die erfindungsgemässe Innenschale 1 ist in den Innenhohlraum einer Aussenschale 2 einführbar und mit dem Befestigungselement 8 an der Aussenschale 2 befestigbar. Dabei werden die Abstützbereiche 6 der Innenschale 1 ungefähr in die Nut 11 der Aussenschale 2 eingelegt und daraufhin die Innenschale 1 durch eine Schwenkbewegung in die Aussenschale 2 eingefahren, sodass die Abstützbereiche 6 vollständig in die Nut 11 zu liegen kommen und das Befestigungselement 8 eine wie in Fig. 4 oder Fig. 5 dargestellte Lage einnimmt. Der in Fig. 4 dargestellte Ausschnitt zeigt die Innenschale 1, die in der Aussenschale 2 liegt. Das Befestigungselement 8, das ein Aussengewinde 8a aufweist, wurde vor der Schwenkbewegung in die Durchbrechung 7 der Innenschale 1 eingeschraubt. Dabei ist die Geometrie der Nut 11, der durch die Abstützbereiche 6 definierte Schwenkpunkt der Innenschale 1 sowie die Ausrichtung und die Lage des Befestigungselementes 8 derart gewählt, dass sich die Innenschale 1 mit der dargestellten Lage des Befestigungselementes 8 in die Aussenschale 2 einschwenken lässt. Im dargestellten Ausführungsbeispiel weicht die Verlaufsrichtung 12 der Durchbrechung 7 um einen Winkel delta, der bis zu wenigen Winkelgraden betragen kann, von einer Parallelen 10a ab. Die Parallele 10a verläuft parallel zur Achsrichtung 10. Weiter ergibt sich zwischen der Verlaufsrichtung 11a der Nut 11 und einer Parallelen 12a zur Verlaufsrichtung 12 ein Winkel gamma. Der Winkel gamma bewirkt, dass sich der Zwischenraum zwischen Innenschale 1 und Aussenschale 2 in Richtung der Anzugsrichtung 13 verengt, sodass sich das Befestigungselement 8, wenn es in Richtung 13 angezogen wird, mit dem Kopf 9 zwischen Innenschale 1 und Aussenschale 2 verkeilt. Dabei wird zwischen dem Innengewinde 7a und dem Aussengewinde 8a vorzugsweise ein Spiel vorgesehen, derart, dass beim Anziehen des Befestigungselementes 8 der Kraftübertragungsbereich 9b an eine Begrenzungsfläche 1i der Innenschale 1 zu liegen kommt und der Kraftübertragungsbereich 9a in die Nut 11 der Aussenschale 2 zu liegen kommt. Somit liegt die Innenschale 1 verkeilt in der Aussenschale 2, indem das Befestigungselement 8 insbesondere radial zur Achsrichtung 10 eine Kraft zwischen Innenschale 1 und Aussenschale 2 überträgt, und indem das Befestigungselement 8 die Innenschale 1 in Verlaufsrichtung 12 in die Aussenschale 2 drückt.

Für ein sicheres Verkeilen der Innenschale 1 in der Aussenschale 2 sind mindestens drei Wirkverbindungen zwischen den beiden Schalen notwendig. In Fig. 1a sind die drei Wirkverbindungen, zwei Abstützbereiche 6 sowie ein Befestigungselement 8 an der Stelle der Durchbrechung 7, gleichmässig über den Umfang verteilt. Die Wirkverbindungen können auch anders entlang dem Umfang verteilt sein. Insbesondere können entlang dem Umfang auch mehr als zwei Abstützbereiche 6 angeordnet sein, oder es kann ein Abstützbereich 6 mit zwei oder mehr Befestigungselementen 8 kombiniert sein, oder es können drei oder mehr Befestigungselemente 8 vorhanden sein. .

Fig. 5 zeigt eine weitere geometrische Anordnung des Befestigungselementes 8 zwischen der Innenschale 1 und der Aussenschale 2. Zwischen der Verlaufsrichtung 12a der Druchbrechung 7 und der Verlaufsrichtung 11a der Nut 11 besteht ein Winkel gamma. Der Winkel zwischen der Achsrichtung 10 und der Verlaufsrichtung 12 ist grösser als der Winkel zwischen der Achsrichtung 10 und einer Seitenfläche 11a der Nut 11 der Aussenschale 2. Der Zwischenraum zwischen Innenschale 1 und Aussenschale 2 verengt sich somit in Richtung der Anzugsrichtung 13, sodass sich das Befestigungselement 8, wenn es in Richtung 13 angezogen wird, mit dem Kopf 9 zwischen der Begrenzungsfläche 1i der Innenschale 1 und der Nut 11 der Aussenschale 2 verkeilt. Dabei wird zwischen dem Innengewinde 7a und dem Aussengewinde 8a vorzugsweise ein Spiel vorgesehen, derart, dass beim Anziehen des Befestigungselementes 8 der Kraftübertragungsbereich 9b an die Begrenzungsfläche 1i der Innenschale 1 zu liegen kommt und der Kraftübertragungsbereich 9a an die Nut 11 der Aussenschale 2 zu liegen kommt. Somit liegt die Innenschale 1 verkeilt in der Aussenschale 2, indem das Befestigungselement 8 insbesondere radial zur Achsrichtung 10 eine Kraft zwischen Innenschale 1 und Aussenschale 2 überträgt.
Fig. 5 zeigt weiter, dass die Innenschale 1 am Pol 4 durch einen zylinderförmigen Fortsatz 4a in einer Ausnehmung 2d der Aussenschale 2 geführt sein kann und dass die Innenschale 1 im Bereich des Pols 4 einen Abstützbereich 5 aufweisen kann, um eine Wirkverbindung zwischen Innenschale 1 und Aussenschale 2 im Bereich des Poles 4 zu ermöglichen.

In der Anordnung gemäss Fig. 5 ist der Winkel gamma sowie die Form des Kopfes 9 derart wählbar, dass sich im Bereich der Durchbrechung 7 der Abstand zwischen der Innenschale 1 und der Aussenschale 2 stufenlos einstellen lässt. In der Darstellung gemäss Fig. 5 liegen Innen- und Aussenschale übereinander. Wird das Befestigungselement 8 vertieft in Anzugsrichtung 13 eingedreht, so kann dies abhängig vom Winkel gamma und der Oberflächenbeschaffenheit von Aussenschale 2 und Kopf 9 bewirken, dass sich dadurch der Abstand zwischen Innen- und Aussenschale vergrössert und somit einstellen lässt.

In der Anordnung gemäss Fig. 5 lässt sich ebenfalls ein Befestigungselement 8 gemäss Fig. 3b anwenden, indem die Durchbrechung 7 ohne Innengewinde 7a aufgeführt ist, sodass das Befestigungselement 8, ähnlich einem Nagel, zwischen Innenschale 1 und Aussenschale 2 einschlagbar ist, bis sich die Kraftübertragungsbereiche 9a, 9b im sich verjüngenden Zwischenraum verkeilen. Dabei kann der Kopf 9 ungefähr den gleichen Durchmesser aufweisen wie das Befestigungselement 8, sodass das Befestigungselement 8 nach dem zusammenfügen der beiden Schalen 1, 2 in die Durchbrechung 7 einführbar ist.

Für das Befestigungselement 8 sind eine Vielzahl von Ausführungsformen möglich, um eine Verkeilung zwischen Innenschale 1 und Aussenschale 2 zu bewirken. So ist die in Fig. 3c dargestellte Ausführungsform eines Kopfes 9 eines Befestigungselementes 8 als ein Beispiel aus einer Vielzahl möglicher Formen zu verstehen, die bewirkt, dass durch eine Drehbewegung des Befestigungselementes 8 die Kraftübertragungsbereiche 9a, 9b gegen die Innenschale 1 beziehungsweise gegen die Aussenschale 2 gedrückt werden, und sich somit eine Verkeilung der Innenschale 1 in der Aussenschale 2 ergibt.

## Patentansprüche

1. Innenschale (1) für eine Hüftgelenkpfanne (3), wobei die Innenschale (1) mit einer metallischen Aussenschale (2) zu einer Hüftgelenkpfanne (3) zusammensetzbar ist, und wobei die Innenschale (1) als Halbkugelschale ausgebildet ist, die einen Pol (4) und eine durch den Pol (4) verlaufende Achsrichtung (10) aufweist,
dadurch gekennzeichnet, dass die Innenschale (1) aus Metall gefertigt ist, dass die Innenschale (1) an ihrer Aussenfläche (1a) im Bereich des Äquators mindestens zwei über den Umfang verteilte Abstützbereiche (6) aufweist, die radial zur Achsrichtung (10) die Aussenfläche (1a) überragen, derart, dass die Abstützbereiche (6) bei zusammengesetzter Hüftgelenkpfanne (3) in einer Nut (11) der Innenfläche (2b) der Aussenschale (2) aufliegen können, dass die Innenschale (1) im Bereich des Äquators eine Durchbrechung (7) mit einer Verlaufsrichtung (12) aufweist, dass die Durchbrechung (7) zur Aufnahme eines Befestigungselementes (8) dient, und dass bei einer in die Aussenschale (2) eingefügten Innenschale (1) das Befestigungselement (8) derart betätigbar ist, dass das Befestigungselement (8) eine Wirkverbindung zur Innenfläche (2b) der Aussenschale (2) erzeugen kann, um die Innenschale (1) im äquatorialen Bereich durch mindestens drei Wirkverbindungen in der Nut (11) der Aussenschale (2) zu verkeilen.

2. Innenschale (1) nach Anspruch 1, dadurch gekennzeichnet, dass der Abstützbereich (6) durch ein Befestigungselement (8) mit Kopf (9) gebildet ist, wobei die Innenschale (1) zur Aufnahme des Befestigungselementes (8) eine Durchbrechung (7) aufweist.

3. Innenschale (1) nach Anspruch 1 oder 2, dadurch gekennzeichnet, dass die Innenschale (1) an ihrer Aussenfläche (1a) im Bereich des Poles (4) einen Abstützbereich (5) aufweist.

4. Innenschale (1) nach einem der Ansprüche 1 bis 3, dadurch gekennzeichnet, dass die Innenschale (1) am Pol (4) einen in Achsrichtung (10) verlaufenden, zylinderförmigen Fortsatz (4a) aufweist.

5. Innenschale (1) nach einem der Ansprüche 1 bis 4, dadurch gekennzeichnet, dass das Befestigungselement (8) ein Aussengewinde (8a) aufweist, das in ein Innengewinde (7a) der Durchbrechung (7) greift.

6. Innenschale (1) nach einem der Ansprüche 1 bis 4, dadurch gekennzeichnet, dass die Beführungsflächen zwischen Befestigungselement (8) und Durchbrechung (7) flächig ausgebildet sind.

7. Innenschale (1) nach einem der Ansprüche 1 bis 6, dadurch gekennzeichnet, dass der Winkel zwischen der Achsrichtung (10) und der Verlaufsrichtung (12) grösser ist als der Winkel zwischen der Achsrichtung (10) und einer Seitenfläche (11a) der Nut (11) der Aussenschale (2), sodass das in Verlaufsrichtung (12) in Richtung (13) der Aussenschale (2) bewegte Befestigungselement (8) mit dessen Kopf (9) an die Seitenfläche (11a) zu liegen kommt.

8. Innenschale (1) nach einem der Ansprüche 1 bis 5, dadurch gekennzeichnet, dass der Winkel zwischen der Achsrichtung (10) und der Verlaufsrichtung (12) kleiner ist als der Winkel zwischen der Achsrichtung (10) und einer Seitenfläche (11a) der Nut (11) der Aussenschale (2), sodass das in Verlaufsrichtung (12) in zur Aussenschale (2) entgegengesetzter Richtung (13) bewegte Befestigungselement (8) mit dessen Kopf (9) an die Seitenfläche (11a) zu liegen kommt.

9. Innenschale (1) nach einem der Ansprüche 1 bis 8, dadurch gekennzeichnet, dass zwischen dem Befestigungselement (8) und der Durchbrechung (7) ein Spiel besteht, damit das Befestigungselement (8) mit Kopf (9) im befestigten Zustand eine Wirkverbindung zwischen der Seitenfläche (11a) der Nut (11) und der Aussenfläche (1a, 1i) der Innenschale (1) eingehen kann.

10. Verfahren zum Verbinden einer metallischen Aussenschale (2) mit einer Innenschale (1) nach einem der Ansprüche 1 bis 9, dadurch gekennzeichnet, dass das Befestigungselement (8) in die Durchbrechung (7) eingeführt wird, dass die Abstützbereiche (6) der Innenschale (1) in eine Nut (11) der Innenfläche (2b) der Aussenschale (2) eingeführt werden, dass die Innenschale (1) mit einer Schwenkbewegung in die Aussenschale (2) geschwenkt wird, dass die Lage des Befestigungselementes (8) in Verlaufsrichtung (12) der Druchbrechung (7) verändert wird, um eine Wirkverbindung zwischen der Nut (11) und der Aussenfläche (1a,1i) der Innenschale (1) zu erzielen, sodass die Innenschale (1) über mindestens zwei Abstützbereiche (6) und ein Befestigungselement (8) in der Nut (11) der Aussenschale (2) verkeilt wird.

11. Verfahren nach Anspruch 10, dadurch gekennzeichnet, dass der Abstand zwischen Innenschale (1) und Aussenschale (2) durch die Positionierung des Befestigungselementes (8) in Verlaufsrichtung (12) eingestellt wird.

12. Metallische Hüftgelenkpfanne (3) mit einer Innenschale (1) nach einem der Ansprüche 1 bis 9 und mit einer metallischen Aussenschale (2), die eine Nut (11) in der Innenfläche (2b) aufweist.

## Claims

1. Inner shell (1) for a hip joint socket (3), wherein the inner shell (1) can be assembled with a metallic outer shell (2) to form a hip joint socket (3), and wherein the inner shell (1) is formed as a hemi-spherical shell having a pole (4) and an axial direction (10) extending through the pole (4), characterized in that the inner shell (1) is made of metal, in that the inner shell (1) has, in the region of the equator, at least two support regions (6) on its outer surface (1a) distributed over the circumference, which extend radially to the axial direction (10) beyond the outer surface (1a) in such a way that, in the assembled hip joint socket (3), the support regions (6) can seat in a groove (11) of the inner surface (2b) of the outer shell (2), in that the inner shell (1) has an aperture (7) in the region of the equator with a direction of extension (12), in that the aperture (7) serves for the receipt of a fastener element (8), and in that, with an inner shell (1) inserted into the outer shell (2), the fastener element (8) is actuatable in such a manner that the fastener element (8) can produce an effective connection to the inner surface (2b) of the outer shell (2) in order to wedge the inner shell (1) in the groove (11) of the outer shell (2) in the equatorial region by means of at least three effective connections.

2. Inner shell (1) in accordance with claim 1, characterized in that the support region (6) is formed by a fastener element (8) with a head (9), wherein the inner shell (1) has an aperture (7) for the receipt of the fastener element (8).

3. Inner shell (1) in accordance with claim 1 or 2, characterized in that the inner shell (1) has a support region (5) at its outer surface (1a) in the region of the pole (4).

4. Inner shell (1) in accordance with one of the claims 1 to 3, characterized in that the inner shell (1) has a cylindrical protuberance (4a) at the pole (4) which extends in the axial direction (10).

5. Inner shell (1) in accordance with one of the claims 1 to 4, characterized in that the fastener element (8) has an outer thread (8a) which engages in an inner thread (7a) of the aperture (7).

6. Inner shell (1) in accordance with one of the claims 1 to 4, characterized in that the guide surfaces between the fastener element (8) and the aperture (7) are areally formed.

7. Inner shell (1) in accordance with one of the claims 1 to 6, characterized in that the angle between the axial direction (10) and the direction of extension (12) is larger than the angle between the axial direction (10) and a lateral surface (11a) of the groove (11) of the outer shell (2), so that the fastener element (8), when moved along the direction of extension (12) in the direction (13) of the outer shell (2), comes to lie with its head (9) on the lateral surface (11a).

8. Inner shell (1) in accordance with one of the claims 1 to 5, characterized in that the angle between the axial direction (10) and the direction of extension (12) is smaller than the angle between the axial direction (10) and a lateral surface (11a) of the groove (11) of the outer shell (2), so that the head (9) of the fastener element (8), when moved along the direction of extension (12) in a direction (13) away from the outer shell (2), comes to lie on the lateral surface (11a).

9. Inner shell (1) in accordance with one of the claims 1 to 8, characterized in that play exists between the fastener element (8) and the aperture (7) so that, in the fastened state, the fastener element (8) with head (9) is able to enter into an effective connection between the lateral surface (11a) of the groove (11) and the outer surface (1a, 1i) of the inner shell (1).

10. Method for the connection of a metallic outer shell (2) with an inner shell (1) in accordance with one of the claims 1 to 9, characterized in that the fastener element (8) is guided into the aperture (7), in that the support regions (6) of the inner shell (1) are introduced into a groove (11) of the inner surface (2b) of the outer shell (2), in that the inner shell (1) is pivoted into the outer shell (2) with a pivotal movement, in that the position of the fastener element (8) in the direction of extension (12) of the aperture (7) is changed in order to achieve an effective connection between the groove (11) and the outer surface (1a, 1i) of the inner shell (1), so that the inner shell (1) is wedged in the groove (11) of the outer shell (2) via at least two support regions (6) and one fastener element (8).

11. Method in accordance with claim 10, characterized in that the spacing between the inner shell (1) and the outer shell (2) is adjusted in the direction of extension (12) via the positioning of the fastener element (8).

12. Metallic hip joint socket (3) with an inner shell (1) in accordance with one of the claims 1 to 9 and with a metallic outer shell (2), which has a groove (11) in the inner surface (2b).

## Revendications

1. Coque intérieure (1) pour une cupule acétabulaire (3), la coque intérieure (1) pouvant être assemblée avec une coque extérieure métallique (2) pour former une cupule acétabulaire (3), la coque intérieure (1) étant réalisée sous forme de coque demi-sphérique qui présente un pôle (4) et une direction d'axe (10) passant à travers le pôle (4), caractérisée en ce que la coque intérieure (1) est réalisée en métal, en ce que la coque intérieure (1) présente sur sa surface extérieure (1a) au voisinage de l'équateur, au moins deux zones d'appui (6) réparties sur le pourtour qui font saillie radialement à la direction d'axe (6) sur la surface extérieure (1a) de telle sorte que les zones d'appui (6), lorsque la cupule acétabulaire (3) est assemblée, peuvent reposer dans une rainure (11) de la surface intérieure (2b) de la coque extérieur (2), en ce que la coque intérieure (1), au voisinage de l'équateur, présente un perçage (7) avec une direction d'extension (12), en ce que le perçage (7) sert à recevoir un élément de fixation (8) et en ce que dans le cas d'une coque intérieure (1) insérée dans la coque extérieure (2), l'élément de fixation (8) peut être actionné de façon que l'élément de fixation (8) puisse établir une liaison active avec la surface intérieure (2b) de la coque extérieure (2) pour coincer la coque intérieure (1) dans la zone équatoriale par au moins trois liaisons actives dans la rainure (11) de la coque extérieure (2).

2. Coque intérieure (1) selon la revendication 1, caractérisée en ce que la zone d'appui (6) est formée par un élément de fixation (8) avec une tête (9), la coque intérieure (1), pour la réception de l'élément de fixation (8), présentant un perçage (7).

3. Coque intérieure (1) selon la revendication 1 ou 2, caractérisée en ce que la coque intérieure (1) présente à sa surface extérieure (1a) au voisinage du pôle (4) une zone d'appui (5).

4. Coque intérieure (1) selon l'une des revendications 1 à 3, caractérisée en ce que la coque intérieure (1) présente au pôle (4) un prolongement cylindrique (4a) s'étendant dans la direction d'axe (10).

5. Coque intérieure (1) selon l'une des revendications 1 à 4, caractérisée en ce que l'élément de fixation (8) présente un filetage extérieur (8a) qui s'engage dans un filetage intérieur (7a) du perçage (7).

6. Coque intérieure (1) selon l'une des revendications 1 à 4, caractérisée en ce que les surfaces de contact entre l'élément de fixation (8) et le perçage (7) sont réalisées de manière plane.

7. Coque intérieure (1) selon l'une des revendications 1 à 6, caractérisée en ce que l'angle entre la direction d'axe (10) et la direction d'extension (12) est plus grand que l'angle entre la direction d'axe (10) et une surface latérale (11a) de la rainure (11) de la coque extérieure (2) de telle sorte que l'élément de fixation (8) déplacé dans la direction d'extension (12) en direction (13) de la coque extérieure (2) s'applique avec sa tête (9) à la surface latérale (11a).

8. Coque intérieure (1) selon l'une des revendications 1 à 5, caractérisée en ce que l'angle entre la direction d'axe (10) et la direction d'extension (12) est plus petit que l'angle entre la direction d'axe (10) et une surface latérale (11a) de la rainure (11) de la coque extérieure (2) de telle sorte que l'élément de fixation (8) déplacé dans la direction d'extension (12) dans la direction (13) opposée à la coque extérieure (2) s'applique avec sa tête (9) à la surface latérale (11a).

9. Coque intérieure (1) selon l'une des revendications 1 à 8, caractérisée en ce qu'il existe entre l'élément de fixation (8) et le perçage (7) un jeu pour que l'élément de fixation (8) avec la tête (9), puisse établir, à l'état fixé, une liaison active entre la surface latérale (11a) de la rainure (11) et la surface extérieure (1a, 1i) de la coque intérieure (1).

10. Procédé pour relier une coque extérieure métallique (2) à une coque intérieure (1) selon l'une des revendications 1 à 9, caractérisé en ce que l'élément de fixation (8) est inséré dans le perçage (7), en ce que les zones d'appui (6) de la coque intérieure (1) sont insérées dans une rainure (11) de la surface intérieure (2b) de la coque extérieure (2), en ce que la coque intérieure (1) est pivotée par un mouvement de pivotement dans la coque extérieure (2), en ce que la position de l'élément de fixation (8) est modifiée dans la direction d'extension (12) du perçage (7) pour obtenir une liaison active entre la rainure (11) et la surface extérieure (1a, 1i) de la coque intérieure (1) de telle sorte que la coque intérieure (1) est coincée par au moins deux zones d'appui (6) et un élément de fixation (8) dans la rainure (11) de la coque extérieure (2).

11. Procédé selon la revendication 10, caractérisé en ce que l'écart entre la coque intérieure (1) et la coque extérieure (2) est réglé par le positionnement de l'élément de fixation (8) dans la direction d'extension (12).

12. Cupule acétabulaire métallique (3) avec une coque intérieure (1) selon l'une des revendications 1 à 9, et avec une coque extérieure métallique (2) qui présente une rainure (11) dans la surface intérieure (2b).
